(19) **Europäisches Patentamt**
**European Patent Office**
**Office européen des brevets**

(11) Publication number : **0 425 643 B1**

# (12) EUROPEAN PATENT SPECIFICATION

(45) Date of publication of patent specification :
**16.03.94 Bulletin 94/11**

(21) Application number : **90907705.9**

(22) Date of filing : **03.05.90**

(86) International application number :
**PCT/US90/02463**

(87) International publication number :
**WO 90/13531 15.11.90 Gazette 90/26**

(51) Int. Cl.$^5$ : **C07C 43/178,** C07C 43/15,
C07C 43/215, C07C 47/575,
C07C 59/60, C07C 65/28,
C07C 69/606, C07C 69/92,
C07H 15/10, C07C 41/06

(54) OCTADIENYL ETHERS AND A PROCESS FOR MAKING OCTADIENYL ETHERS.

(30) Priority : **03.05.89 US 347096**
**02.05.90 US 517990**

(43) Date of publication of application :
**08.05.91 Bulletin 91/19**

(45) Publication of the grant of the patent :
**16.03.94 Bulletin 94/11**

(84) Designated Contracting States :
**AT BE CH DE DK ES FR GB IT LI LU NL SE**

(56) References cited :
**US-A- 3 530 187**
**US-A- 4 006 192**
**JOURNAL OF MEDICINAL CHEMISTRY, vol.
31, no. 1, January 1988, American Chem. Soc.,
US; H. NAKAI et al, "New potent antagonists
of leukotrienes C4 and D4. 1. Synthesis and
structure-activity relationships", pages 84-91
JOURNAL OF ORGANOMETALLIC CHEMIS-
TRY, vol. 137, no. 3, 20 September 1977,
Elsevier Sequoia S.A., Lausanne, CH; R.
KLÜter et al, "Die Cooligomerisierung von
Butadien mit alpha-Hydroxycarbonsäurees-
tern", pages 309-314
SYNTHESIS, no. 5, May 1977; H. YAGI et al,
"Synthesis of 2,6-dimethyl-1,3-trans,7-octat-
riene (head-to-tail isoprene dimer) by a tem-
perature-controlled two-stayge reaction",
pages 334-335**

(73) Proprietor : **HENKEL RESEARCH
CORPORATION
2330 Circadian Way
Santa Rosa California 95407 (US)**

(72) Inventor : **GRUBER, Bert
Albert-Schlangen-Strasse 28
D-5012 Bedburg (DE)**
Inventor : **WEESE, Kenneth, J.
1026 Baird Road
Santa Rosa, CA 94121 (US)**
Inventor : **HOAGLAND, Steven, M.
739 36th Avenue, nr. 204
San Francisco, CA 94121 (US)**
Inventor : **MUELLER, Hans-Peter
Jahnstrasse 1
D-4040 Neusse 1 (DE)**
Inventor : **HILL, Karl-Heinz
Am Hasenbusch 1
D-4006 Erkrath (DE)**
Inventor : **BEHR, Arno
Marbacher Strasse 7
D-4000 Düsseldorf-Benrath 13 (DE)**

(74) Representative : **von Kreisler, Alek,
Dipl.-Chem. et al
Patentanwälte von Kreisler-Selting-Werner
Postfach 10 22 41
D-50462 Köln (DE)**

## Description

### 1. Field of the Invention

This invention relates to a process for making alkadienyl ethers.

### 2. Statement of Related Art

It is known in the art that conjugated dienes can telomerize with alcohols to give 1,7- and 2,7-alkadienyl ethers. The following references exemplify extensive patent and other literature that relate to such telomerization reactions and compounds prepared by such reactions: U.S. Patent Nos. 3,489,813; 3,499,042; 3,670,032; 3,769,352; 3,792,101; 3,887,627; 3,891,684; 3,923,875; 3,992,456; 4,006,192; 4,142,060; 4,146,738; 4,196,135; 4,219,677; 4,260,750; 4,356,333; 4,417,079; 4,454,333; 4,515,711; 4,522,760; and 4,642,392. British Patent Nos. 1,248,592; 1,248,593; 1,354,507; 2,054,394; and 2,114,974. German Patent Nos. 1,807,491; 2,154,370; and 2,505,180. Japanese Patent Nos. 72,020,604; 47,031,906; 48,039,413; 73,042,606; 73,003,605; 49,031,965; 49,048,613; 49,125,313; 74,046,286; 50,157,301; 51,008,206; 51,142,532; 51,149,206; and 51,007,426. Literature articles: Behr, Organometallics 5, 514-8 (1986) Jolly, Organometallics 5, 473-81 (1986) Dzhemilev, Zh. Org. Khim. 22 (8), 1591-7 (1986) Gaube, J. Prakt. Chim. 327 (4), 643-8 (1985) Jolly, Organometallics 4, 1945-53 (1985) Bochmann, J. Molec. Catalysis 26, 79-88 (1984) Behr, Aspects of Homogeneous Catalysis 5, 5-58 (1984) Gaube, J. Prakt. Chem. 326, (6) 947-54 (1984) Behr, Chem. Ber. 116, 862-73 (1983) Groult, Tetrahedron 39, (9) 1543-50 (1983) Teranishi, J. Org. Chem. 46, 2356-62 (1981) Dzhemilev, Izv. Akad. Nauk. SSSR, Ser. Khim. 8, 1837-425 (1981) Keim, J. Molec. Catalysis 10, 247-252 (1981) Dzhemilev, Zh. Org. Khim. 16 (6), 1157-61 (1980) Yoshida, Tetr. Letters 21, 3787-90 (1980) Tsuji, Pure & Appl. Chem. 51, 1235-41 (1979) Tsuji, Adv. in Organometallic Chem. 17, 141-93 (1979) Singer, J. Organomet. Chem. 137 (3), 309-14 (1977) Chauvin, Tet. Letters 51, 4559-62 (1975) Chauvin, Bull. Soc. Chim. Fr. 652-6 (1974) Beger, J. Prakt. Chem. 315 (6), 1067-89 (1973) Baker, Chemical Reviews 73 (5), 503-9 (1973) Tsuji, Accounts Chem. Res. 6 (1), 8-15 (1973) Smutny, Annals N.Y. Acad. Sci. 214, 124-142 (1973) Chauvin, Tetr. Letters 51, 4559-62 (1973) Rose, J. Organometallic Chem. 49, 473-6 (1973) Smutny, ACS, Div. Petr. Chem., Prepn. 14 (2), B100-11 (1969) Takahashi, Bull. Chem. Soc. Japan 41, 254-5 (1968) Takahashi, Bull. Chem. Soc. Japan 41, 454-60 (1968) Smutny, J. Am. Chem. Soc. 89, 6793-4 (1967) Takahashi, Tetr. Letters (26), 2451-3 (1967).

U.S. patent No. 4,006,192 discloses the preparation of the di- and tri-1-(2,7-octadienyl) ethers of trimethylolpropane; by telomerisation of butadiene with trimethylpropane in the presence of a palladium catalyst and triphenylphosphine in an autoclave; British patent application No. 2,054,394 discloses the preparation of the di-1-(2,7-octadienyl) ether of ethylene glycol; Zh. Org. Khim. 16 (6), 1157-61 (1980) discloses the preparation of the mono- and di-1-(2,7-octadienyl) ethers of 1,4-butanediol, 1,2-propylene glycol, glycerol, and the mono-1-(2,7-octadienyl) ether of 2-hydroxyethoxyethanol; British patent No. 1,354,507 discloses the preparation of 1-(2,7-octadienyl) ether of methanol; J. Prakt. Chem., 315, 1067-76 (1973) discloses the preparation of 1-(2,7-octadienyl) ethers of n-butanol, n-hexanol, n-octanol, n-decanol, and n-dodecanol; Japanese patent No. JP 49,031,965 discloses the mono-1-(2,7-octadienyl) ether of ethylene glycol.

These alkadienyl ethers are most readily prepared by the telomerization of a conjugated alkadiene by alcohols in the presence of palladium catalysts. However, the development of economically practical telomerization processes has not been achieved primarily because of the cost of the palladium catalysts. The prior art contains many examples of attempts to deal with the catalyst cost problem. For example, U.S. patent No. 4,642,392 discloses a catalyst recovery method which employs the use of a high boiling reaction solvent which allows for the distillation of the product telomer leaving behind catalyst solution which can be reused many times with, presumably, minimum palladium loss. Other catalyst recovery methods are disclosed in U.S. patent Nos. 4,454,333; 4,552,760; 4,260,750; 4,219,667; 4,142,060; 4,417,079; 4,356,333; Japanese patent No. 50,157,301; 51,149,206.and British patent No. 2,054,394. All of the above catalyst recovery schemes require additional process operations which add to the cost of the final product.

The use of low palladium levels in the telomerization of conjugated dienes is disclosed in British patent No. 2,114,974. This patent teaches that when 1,4-butanediol, 1,5-pentanediol, and 1,6-hexanediol telomerized with butadiene, about a 61% yield of monoether based on butadiene can be realized by using a molar ratio of catalyst/diene equal to about 1/20,600. The patent also discloses that a large stoichiometric excess of diol is also necessary in order to obtain high yields of the desired monoether product. For best yields of monoether, the above patent also teaches that the optimum catalytic effect is obtained by combining the palladium catalyst with a nickel(II) compound and a base such as a quaternary ammonium hydroxide. The patent also discloses a method of removing the palladium catalyst with ion exchange resins from the reaction mixture after the reaction has been completed. The palladium levels are obviously not low enough to preclude the recovery step; an operation which the present invention eliminates. U.S. Patent No. 3,746,749 discloses that octadienyl esters of adipic acid can be prepared in 86% yield by employing a molar ratio of Pd/Acid/Butadiene equal to 1/12,300/56,000 and octadienyl esters of fumaric acid can be prepared in 67% yield by employing a molar ratio of Pd/Acid/Butadiene equal to 1/60,000/28,000. However, these low palladium levels are used in conjunction with from 0.1 to 10 moles of an alkali metal salt of a carboxylic acid/mole of carboxylic acid.

The process of the present invention overcomes the disadvantages of the prior art processes by utilizing catalyst amounts low enough to allow it to be economically feasible to permit the catalyst to remain in the final reaction products without the need for recovery. At the same time, the yield of dienyl ether product is high while that of the dimerized diene side product is low at these extremely low catalyst concentrations.

In detail the present invention relates to a process for the telomerisation of conjugated dienes with an alcohol reactant comprising the steps of:

(1) reacting

   (a)

      - as the alcohol reactant at least one polyol containing two -$CH_2OH$ groups separated by at least two carbon atoms, or

      - as the alcohol reactant glycerol (1,2,3-propanetriol) or

      - an alcohol reactant, which is not soluble in the reaction mixture and

   (b) at least one conjugated diene

      - in the presence of a palladium catalyst which is present in a mole ratio of catalyst to diene of 1:10 000 to 1:75 000 and

      - in the presence of from 10 to 50 % by weight of at least one saturated aliphatic secondary or tertiary alcohol solvent, and

(2) isolating the telomerisation reaction product from said reaction mixture.

The process of the present invention rests on the discovery that conjugated dienes can be telomerized with alcohols to form 1-substituted dienyl ethers in high yields in the presence of very low levels of palladium catalyst.

According to the present invention low levels of palladium catalyst to be effective in producing high yields of the desired alkadienyl ether products.

In the telomerization processes of the prior art relatively large concentrations of palladium catalysts are required in order to realize practical yields of product. Since the catalysts are so expensive, they have to be recovered in order to make the processes economically viable. Because these recovery operations add to the costs of the telomerization processes, the commercial production of dienyl ethers has not been widespread. The process of the present invention eliminates the need for using prohibitively high palladium catalyst levels and for costly catalyst recovery operations. The process of the present invention is a method of telomerizing conjugated dienes with alcohols in the presence of very low levels of palladium catalyst relative to the amount of diene used in the reaction. The process of the present invention is carried out by reacting one or more polyols with one or more conjugated dienes in the presence of a catalyst effective amount of a palladium catalyst. The amount of catalyst is a function of the amount of conjugated diene and is expressed as the molar ratio of catalyst:diene. The effective amount of catalyst of the present invention is preferably in the range of from 1:25,000 to 1:75,000.

The polyols which can be employed in the process of the present invention include compounds of the formula 1,4-butanediol, 1,5-pentanediol, 1,6-hexanediol, 1,10-decanediol, 1,12-dodecanediol and glycerol (1,2,3-propanetriol). The preferred polyol in these cases is glycerol.

The conjugated dienes that can be employed in the process of the present invention include branched- or straight-chain aliphatic conjugated dienes containing from 4 to 20 carbon atoms, or cyclic dienes containing from 6 to 8 carbon atoms, and which may optionally be substituted with one or more inert groups such as $C_1$-$C_8$ alkyl groups, phenyl, cyclohexyl, nitro, oxo, alkoxycarbonyl having 1 to 4 carbon atoms in the alkyl group, or halogen such as fluorine or chlorine wherein the halogen is in a position inert to the process conditions, for example, chloroprene. Other conjugated dienes that can be used in the process of this invention include 1,3-butadiene, dimethylbutadiene, isoprene, piperylene, 1,3-hexadiene, 2,4-hexadiene, chloroprene, 1-cyclohexyl-1,3-butadiene, 1-phenyl-1,3-butadiene, 2,4-octadiene, piperylene, 2-methyl-2,4-pentadiene, 1,3-cyclohexadiene, and 1,3-cyclooctadiene. The preferred conjugated diene is 1,3-butadiene.

The process of the present invention is carried out in cases where the alcohol reactant is a polyol having two -$CH_2OH$ groups separated by at least two carbon atoms, or in cases where the alcohol reactant, is not soluble in the reaction mixture, whereby it is necessary to add from 10% to 50% by weight of at least one saturated aliphatic secondary or tertiary alcohol solvent in order for the low levels of palladium(II) catalyst to be effective in producing high yields of the desired alkadienyl ether products. The secondary or tertiary alcohol solvents are saturated aliphatic alcohols having from 3 to 10 carbon atoms preferably 3 to 4 carbon atoms. The secondary or tertiary alcohol solvents can also contain a primary alcohol group in addition to a secondary or tertiary alcohol group. For example, 1,2-propanediol could be used as the solvent because it contains a secondary alcohol group in addition to the primary alcohol group. The preferred secondary alcohol solvents are isopropyl alcohol (2-propanol) and 1,2-propanediol. The preferred tertiary alcohol solvent is t-butyl alcohol (2-methyl-2-propanol). Mixtures of two or more of such secondary and/or tertiary alcohol solvents can also be used.

The ratio of alcohol reactant to conjugated diene reactant is preferably about two moles of diene per mole of alcohol functionality present in the alcohol. However, if desired, ratios of alcohol equivalents to conjugated diene equivalents of from 0.8-1.5 to 1 can also be employed, or even using a greater excess of alcohol or conjugated diene, but the most advantageous and economical embodiment of the process is when the reactants are approximately equivalent.

The catalyst used in the process is a catalyst system comprised of a palladium compound and a cocatalyst. The palladium catalysts are selected from the group consisting of palladium acetylacetonate [Pd(acac)$_2$], bis(allyl)palladium [Pd(C$_3$H$_5$)$_2$], bis(cyclooctadiene)palladium [Pd(COD)$_2$], palladium chloride (PdCl$_2$), palladium acetate [Pd(OAc)$_2$], and allyl palladium chloride [Pd(C$_3$H$_5$)Cl]. The cocatalysts are selected from the group consisting of trialkylphosphine, triarylphosphine, or triarylphosphite. A mixture of alkyl, aryl, or aryl/alkyl phosphines and phosphites can also be used. Examples of trialkylphosphines include triethylphosphine and tributylphosphine. Examples of triarylphosphines include triphenylphosphine, o-,m-,p-tolylphosphine, 1,2-bis(diphenylphosphino)ethane, and 1,2-bis(di-p-tolylphosphino)ethane. Examples of triarylphosphites include tri(o-tolyl)phosphite, triphenylphosphite, trimethylphosphite, and triethylphosphite. Another suitable catalyst is tetrakis(triphenylphosphine)palladium [Pd(PPh$_3$)$_4$].

The most preferred catalyst systems are complexes of the acetylacetonate of Pd(II) with two equivalents of triphenylphosphine as a ligand, palladium(II) acetate with two equivalents of triphenylphosphine as a ligand, palladium(II) chloride with two equivalents of triphenylphosphine as a ligand, and tetrakis(triphenylphosphine)palladium.

The reaction is achieved without the addition of a catalyst reductant such as triethylaluminum or sodium borohydride, which are often used in the prior art processes. The catalyst used herein is preferably prepared by combining a palladium catalyst with two equivalents of a cocatalyst in the alcohol reactant to be used in the process. The conjugated diene is then added to the above to form a reaction mixture. The preferred amount of the saturated aliphatic secondary or tertiary alcohol solvent to be added must be determined on an individual basis.

The synthesis of the compounds of the present invention as well as the process of the present invention are carried out in any suitable reaction vessel, such as a glass autoclave, steel autoclave, or any other reaction vessel equipped for reactions under pressure.

The reaction temperatures are in the range of from 400C to 100°C preferably in the range of from 60°C to 80°C.

The general procedure for the synthesis of the compounds of the present invention as well as the process of the present invention involves introducing the palladium catalyst-alcohol reactant solution into a reaction vessel along with any necessary saturated aliphatic secondary or tertiary alcohol solvent or mixtures thereof, followed by addition of the diene. The reaction mixture is heated slowly to a reaction temperature of 40°C to 100°C and the reaction is carried out for a period of from 5 to 15 hours. Alternatively, the conjugated diene can be added in increments to the other reaction mixture components after they have been heated to reaction temperature.

The reaction products are isolated by standard techniques such as distillation, crystallization or filtration.

The following examples are meant to illustrate but not limit the invention.

Reference Example Telomerization of 1,3-butadiene with 1,2-propanediol (not according to the invention).

To a 400 ml glass autoclave equipped with a magnetic stir bar, there was added 0.0038 grams (0.0125 mmol) of palladium(II) acetylacetonate, 0.0066 grams (0.025 mmol) of triphenylphosphine, and 47.6 grams (625 mmol) of 1,2-propanediol. The autoclave was connected to a pressure gauge and the assembly was weighed. The autoclave assembly was evacuated and flushed three times with argon after which 70.9 grams (1313 mmol) of 1,3-butadiene was added with a syringe. The autoclave was then reweighed, heated to 70°C and maintained at that temperature for 12 hours while stirring at 500 rpm. The autoclave was then cooled to room temperature, weighed to make sure that no leakage had occurred during the reaction, and vented to release any unreacted 1,3-butadiene. The autoclave assembly was then reweighed to determine the amount of 1,3-butadiene consumed in the reaction. The reaction product was a clear, colorless, homogeneous liquid which contained 69.9% by weight monoether, 8.9% by weight diether, and 7.6% by weight octatriene, and 14.6% by weight 1,2-propanediol.

Example 1. Telomerization of 1,3-butadiene with glycerol in the presence of 2-propanol.

To a 400 ml glass autoclave equipped with a magnetic stir bar, there was added 0.0038 grams (0.0125 mmol) of palladium (II) acetylacetonate, 0.0066 grams (0.025 mmol) of triphenylphosphine, 28.78 grams (313 mmol) of glycerol, and 35 ml of 2-propanol. The autoclave was connected to a pressure gauge and the assembly was weighed. The autoclave assembly was evacuated and flushed three times with argon after which 37.8 grams (700 mmol) of 1,3-butadiene was added with a syringe. The autoclave was then reweighed, heated to 70°C and maintained at that temperature for 12 hours while stirring at 500 rpm. The autoclave was then cooled to room temperature, weighed to make sure that no leakage had occurred during the reaction, and vented to release any unreacted 1,3-butadiene. The autoclave assembly was then reweighed to determine the amount of 1,3-butadiene consumed in the reaction. The reaction product was a clear, slightly colored liquid. The product contained 11.3 % by weight glycerol, 34.0 % by weight glycerol monoether, 21.7 % by weight glycerol diether, 2.1 % by weight glycerol triether, and 2.82% by weight octatriene.

Example 2. Comparative Solvent Effects on the Telomerization of 1,3-butadiene with glycerol

The procedure described in Reference Example was followed in producing the data listed in Table 1. The data shows that isopropanol is the most effective solvent for improving the yield of telomerized product while keeping the amount of octatriene to a minimum. The reaction time is also considerably shorter in the presence of isopropanol.

TABLE 1[a]
SOLVENT EFFECT ON TELOMERIZATION
OF BUTADIENE WITH GLYCEROL

| Solvent | %BD[b] | %Olig[c] | %Telomers | | | Time(hr) |
|---|---|---|---|---|---|---|
| | | | Mono | Di | Tri | |
| NONE | 98 | 7.01 | 1.67 | 71.19 | 8.43 | 14 |
| toluene | 63 | 54.5 | --- | 28.4 | 15.0 | 10 |
| diethylether | 8.0 | 52.8 | 18.2 | 11.5 | 3.0 | 10 |
| THF | 16 | 37.8 | 31.7 | 30.5 | --- | 10 |
| isopropanol[*] | 100 | 3.8 | 50.8 | 36.2 | 4.2 | 1.5 |
| ethyl acetate | 15 | 83.2 | 16.8 | ---- | ---- | 10 |
| acetone | 65 | 10.7 | 27.8 | 47.5 | 10.6 | 10 |
| acetonitrile | 98 | 12.5 | 26.6 | 47.3 | 10.0 | 10 |
| DMSO | 99 | 25.9 | 33.9 | 29.0 | 4.2 | 10 |

a- molar ratio Pd(acac)$_2$:PPh$_3$:butadiene:glycerol = 1:2:4100:2000
b- %BD is the % of butadiene consumed in the reaction.
c- % oligomer is % octatriene
*)according to the present invention

Example 3. Effect of Catalyst Concentration on Telomerization

The use of low concentrations of catalyst is exemplified in the data presented in Table 2. The results which were obtained in a study of the effect of catalyst concentration in the telomerization of 1,3-butadiene with glycerol are illustrative of the process of the present invention.

TABLE 2[1]

EFFECT OF CATALYST CONCENTRATION IN THE TELOMERIZATION OF BUTADIENE WITH GLYCEROL

| Pd: BD[a] | %BD[c] | %Oligomer[b] | %Telomers | | | Time(hr) |
|---|---|---|---|---|---|---|
| | | | Mono | Di | Tri | |
| 1: 9184 | 100 | 4.8 | 54.5 | 32.9 | 2.2 | 2 |
| 1: 17908 | 100 | 5.8 | 49.5 | 38.1 | 3.0 | 5 |
| 1: 31848 | 96 | 5.9 | 49.6 | 34.9 | 2.5 | 7 |
| 1: 56880 | 100 | 7.6 | 48.9 | 33.8 | 2.5 | 10 |
| 1: 66064 | 94 | 8.8 | 54.2 | 31.6 | 1.9 | 20 |
| 1: 84432 | 78 | 10.6 | 60.9 | 23.6 | 1.4 | 25 |

a- BD is butadiene.
b- % oligomer is % octatriene
c- %BD is the % of butadiene consumed in the reaction.
1- temperature = 70°C; solvent - isopropanol

Comparative Example. Solvent Effect on the Telomerization of 1,3-butadiene with 1,4-butanediol.

The procedure of example 2 was followed using the quantities of 1,4-butanediol, isopropyl alcohol (IPA), and 1,3-butadiene indicated in Table 3A. The results which are listed in Table 3B show that the presence of isopropanol in the reaction increases the product yield and the consumption of butadiene. The data in Table 3 also show that efforts to repeat the results disclosed in British Patent No. 2,114,974 (experiments D and E) were not successful, and that the use of isopropyl alcohol as solvent gave markedly enhanced conversions of butadiene and 1,4-butanediol to telomer product.

TABLE 3A

TELOMERIZATION OF 1,4-BUTANEDIOL
RAW MATERIALS USED

| Exp. No. | BD[a] (grams) | Diol[a] (grams) | IPA (grams) | Pd: BD[a] : diol[a] molar ratio | Time (hrs) |
|---|---|---|---|---|---|
| A | 18.2 | 74.1 | 15.7 | 1: 26960: 65840 | 12 |
| B | 13.2 | 52.1 | 15.7 | 1: 19520: 46240 | 13 |
| C | 13.5 | 33.9 | 19.6 | 1: 20000: 30080 | 6 |
| D | 13.7 | 45.5 | 0 | 1: 24190: 48095 | 4 |
| E | 13.6 | 45.3 | 0 | 1: 24000: 47904 | 4 |

TABLE 3B
TELOMERIZATION OF 1,4-BUTANEDIOL
PRODUCT ANALYSES

| Exp. No. | %BD[d] | Remaining[c] diol % | %Olig[b] | %Telomers Mono | Di | Ref |
|---|---|---|---|---|---|---|
| A | 36 | 58.6 | 2.2 | 36.7 | 2.5 | 1 |
| B | 83 | 59.5 | 2.4 | 37.0 | 1.1 | 1 |
| C | 100 | 51.7 | 4.3 | 41.0 | 1.9 | 1 |
| D | 17 | 98.1 | ND | 1.9 | ND | 2 |
| E | 17 | 98.4 | ND | 1.6 | ND | 2 |

a- BD is butadiene, diol is 1,4-butanediol.
b- % oligomer is % octatriene
c- % diol is the percent of the original diol which has not reacted, and remains in the solution.
d- %BD is the % of butadiene consumed in the reaction.
1- catalyst- $Pd(acac)_2$ : 2 $(Ph)_3P$; reaction temperature = 70°C.
2- catalyst- $Pd(OAc)_2(Ph_3P)_2$; 1.7 equiv. $Ni(OAc)_24H_2O$ added; temperature = 75°C as disclosed in British Patent No. 2,114,974.

**Claims**

1. A process for the telomerisation of conjugated dienes with an alcohol reactant comprising the steps of:
    (1) reacting
        (a)
            - as the alcohol reactant at least one polyol containing two -$CH_2OH$ groups separated by at least two carbon atoms, or
            - as the alcohol reactant glycerol (1,2,3-propanetriol) or
            - an alcohol reactant, which is not soluble in the reaction mixture and
        (b) at least one conjugated diene
                - in the presence of a palladium catalyst which is present in a mole ratio of catalyst to diene of 1:10 000 to 1:75 000 and
                - in the presence of from 10 to 50 % by weight of at least one saturated aliphatic secondary or tertiary alcohol solvent, and
        (2) isolating the telomerisation reaction product from said reaction mixture.

2. A process according to claim 1, characterized in that the polyol is 1,4-butanediol, 1,5-pentanediol, 1,6-hexanediol, 1,10-decanediol, 1, 12-dodecanediol.

3. A process of claims 1 or 2 wherein said palladium catalyst is selected from the group consisting of acetylacetonate of Pd(II) with two equivalents of triphenylphosphine as a ligand, palladium(II) acetate with two equivalents of triphenylphosphine as a ligand, palladium(II) chloride with two equivalents of triphenylphosphine as a ligand, and tetrakis(triphenylphosphine)palladium.

4. A process of claims 1 to 3 wherein said palladium catalyst is acetylacetonate of Pd(II) with two equivalents of triphenylphosphine.

5. The process of claims 1 to 4 wherein said mole ratio of catalyst to diene is from 1:25,000 to 1:75,000.

6. The process of claims 1 to 5 wherein said conjugated diene is a branched- or straight-chain aliphatic diene having from 4 to 20 carbon atoms or a cyclic diene having from 5 to 8 carbon atoms.

7. The process of claims 1 to 6 wherein said conjugated diene is 1,3-butadiene, piperylene, isoprene, or mixtures thereof.

8. The process of claims 1 to 7 wherein step (1) is carried out at a temperature of from 40°C to 100°C.

9. The process of claims 1 to 8 wherein said secondary or tertiary alcohol solvent contains from 3 to 10 carbon atoms.

10. The process of claim 9 wherein said secondary alcohol solvent is 2-propanol.

**Patentansprüche**

1. Verfahren zur Telomerisierung konjugierter Diene mit einem Alkohol-Reaktionspartner, umfassend die Schritte
   (1) des Umsetzens
       (a)
           - wenigstens eines, zwei -CH$_2$OH -Gruppen, die durch wenigstens zwei Kohlenstoff-Atome voneinander getrennt sind, enthaltenden Polyols, als dem Alkohol-Reaktionspartner oder
           - von Glycerin (1,2,3-Propantriol) als dem Alkohol-Reaktionspartner oder
           - eines Alkohol-Reaktionspartners, der nicht in der Reaktionsmischung löslich ist, und
       (b) wenigstens eines konjugierten Diens
           - in Gegenwart eines Palladium-Katalysators, der in einem Stoffmengen-Verhältnis Katalysator zu Dien von 1 : 10 000 bis 1 : 75 000 vorhanden ist,
           - in Gegenwart von 10 bis 50 Gew.-% wenigstens eines Lösungsmittels aus einem gesättigten aliphatischen sekundären oder tertiären Alkohol und
   (2) des Isolierens des Telomerisations-Reaktionsprodukts aus der Reaktionsmischung.

2. Verfahren nach Anspruch 1, dadurch gekennzeichnet, daß das Polyol 1,4-Butandiol, 1,5-Pentandiol, 1,6-Hexandiol, 1,10-Decandiol, 1,12-Dodecandiol ist.

3. Verfahren nach den Ansprüchen 1 oder 2, worin der Palladium-Katalysator aus der aus Pd(II)-acetylacetonat mit zwei Äquivalenten Triphenylphosphin als Ligand, Palladium(II)-acetat mit zwei Äquivalenten Triphenylphosphin als Ligand, Palladium(II)-chlorid mit zwei Äquivalenten Triphenylphosphin als Ligand und Tetrakis(triphenylphosphin)palladium bestehenden Gruppe ausgewählt ist.

4. Verfahren nach den Ansprüchen 1 bis 3, worin der Palladium-Katalysator Pd(II)-acetylacetonat mit zwei Äquivalenten Triphenylphosphin ist.

5. Verfahren nach den Ansprüchen 1 bis 4, worin das Stoffmengen-Verhältnis Katalysator zu Dien 1 : 25 000 bis 1 : 75 000 beträgt.

6. Verfahren nach den Ansprüchen 1 bis 5, worin das konjugierte Dien ein verzweigtes oder geradkettiges aliphatisches Dien mit 4 bis 20 Kohlenstoff-Atomen oder ein cyclisches Dien mit 5 bis 8 Kohlenstoff-Atomen ist.

7. Verfahren nach den Ansprüchen 1 bis 6, worin das konjugierte Dien 1,3-Butadien, Piperylen, Isopren oder eine Mischung aus diesen ist.

8. Verfahren nach den Ansprüchen 1 bis 7, worin der Schritt (1) bei einer Temperatur von 40 °C bis 100 °C durchgeführt wird.

9. Verfahren nach den Ansprüchen 1 bis 8, worin das Lösungsmittel aus einem sekundären oder tertiären Alkohol 3 bis 10 Kohlenstoff-Atome enthält.

10. Verfahren nach Anspruch 9, worin das Lösungsmittel aus einem sekundären Alkohol 2-Propanol ist.

**Revendications**

1. Procédé de télomérisation de diènes conjugués par un réactif d'alcool, comprenant les étapes consistant à :

(1) faire réagir

    (a)

        - comme le réactif d'alcool, au moins un polyol contenant deux groupes $CH_2OH$ séparés par au moins deux atomes de carbone, ou

        - comme le réactif d'alcool, du glycérol (1,2,3-propanétriol), ou

        - un réactif d'alcool qui n'est pas soluble dans le mélange de réaction, et

    (b) au moins un diène conjugué

        - en présence d'un catalyseur de palladium qui est présent dans un rapport molaire du catalyseur au diène de 1:10000 à 1:75000, et

        - en présence de 10 % à 50 % en poids d'un solvant d'alcool secondaire ou tertiaire aliphatique saturé , et

(2) isoler le produit de réaction de télomérisation, du mélange de réaction.

**2.** Procédé selon la revendication 1, caractérise en ce que le polyol est du 1,4-butanediol, du 1,5-pentanediol, du 1,6-hexanediol, du 1,10-décanediol, du 1,12-dodécanediol.

**3.** Procédé selon l'une des revendications 1 ou 2, caractérisé en ce que le catalyseur de palladium est choisi dans le groupe comprenant l'acétylacétonate de Pd (II) avec deux équivalents de triphénylphosphine comme liant, l'acétate de palladium (II) avec deux équivalents de triphénylphosphine comme liant, le chlorure de palladium (II) avec deux équivalents de triphénylphosphine comme liant, et le tétrakis (triphénylphosphine) palladium.

**4.** Procédé selon l'une des revendications 1 à 3, caractérisé en ce que le catalyseur de palladium est de l'acétylacétonate de Pd (II) avec deux équivalents de triphénylphosphine.

**5.** Procédé selon l'une des revendications 1 à 4, caractérisé en ce que le rapport molaire du catalyseur au diène, est de 1:25000 à 1:75000.

**6.** Procédé selon l'une des revendications 1 à 5, caractérisé en ce que le diène conjugué est un diène aliphatique à chaîne ramifiée ou droite, comportant de 4 à 20 atomes de carbone, ou un diène cyclique comportant de 5 à 8 atomes de carbone.

**7.** Procédé selon l'une des revendications 1 à 6, caractérisé en ce que le diène conjugué est du 1,3-butadiène, du pipérylène, de l'isoprène, ou des mélanges de ces produits.

**8.** Procédé selon l'une des revendications 1 à 7, caractérisé en ce que l'étape (1) est effectuée à une température de 40°C à 100 °C.

**9.** Procédé selon l'une des revendications 1 à 8, caractérisé en ce que le solvant d'alcool secondaire ou tertiaire contient de 3 à 10 atomes de carbone.

**10.** Procédé selon la revendication 9, caractérisé en ce que le solvant d'alcool secondaire est du 2-propanol.